# EUROPEAN PATENT APPLICATION

(11) **EP 4 635 983 A1**
(43) Date of publication of application: **22.10.2025**
(21) Application number: 24170301.6
(22) Date of filing: 15.04.2024
(51) Int. Cl.: C07K 16/28, A61P 35/00

(54) **A NOVEL ANTIBODY BINDING SPECIFICALLY TO NPTXR AND USE THEREOF**

(71) Applicant: Ymmunobio AG, 4125 Riehen (CH)
(72) Inventor: Rupalla, Dr. Katrin, 4125 Riehen (CH); Schiemann, Dr. Peter, Riehen 4125 (CH); Janicot, Dr. Michel, 1180 Uccle (BE)
(74) Representative: Gulde & Partner

(57) **Abstract**

The present invention is directed to an isolated antibody or antigen binding fragment thereof binding specifically to human NPTXR with the amino acid sequence of SQ ID NO: 1, wherein the isolated antibody or antigen binding fragment comprises three heavy chain complementarity determining regions (HCDR1, HCDR2, and HCDR3), and three light chain complementarity determining regions (LCDR1, LCDR2, and LCDR3), wherein HCDR1 comprises the amino acid sequence of SEQ ID NO: 4; HCDR2 comprises the amino acid sequence of SEQ ID NO: 5; and HCDR3 comprises the amino acid sequence of SEQ ID NO: 6; and wherein LCDR1 comprises the amino acid sequence of SEQ ID NO: 7; LCDR2 comprises the amino acid sequence of SEQ ID NO: 8; and LCDR3 comprises the amino acid sequence of SEQ ID NO: 9.

## Description

### INTRODUCTION:

Stomach cancer is a cancer with the fourth highest number of cases and the second highest cancer-related number of deaths throughout the world (statistics in 2012). Stomach cancer is a common cancer in Asian countries such as Japan, China, Korea, and the like and South America, and globally this cancer type leads to approx. 1.1 Mio new cases per year and roughly 770000 deaths per year (source: globocan 2020). Thus, the incidence rate is high, and stomach cancer is a serious disease that should be overcome with priority.

In Japan, the mortality of stomach cancer tends to decrease because instances of early detection have increased due to spread of cancer screening. However, since substantially no symptom is found in an early-stage stomach cancer, a stomach cancer is still often detected after developed to an advanced stomach cancer, which causes a high mortality. As a primary treatment for an unresectable advanced recurrence stomach cancer, combined therapy of a fluoropyrimidine (fluorouracil or capecitabine) combined with either oxaliplatin or cisplatin is established as a standard therapy globally. For tumours with PD-L1 expression levels by CPS of ≥5 (category 1) additionally an anti-PD-1 antibody is recommended. For tumours expressing HER2, trastuzumab that is an anti-HER2 antibody is a standard therapeutic drug against an HER2 positive case is recommended to be added to standard chemotherapy. Further, as a secondary treatment, ramucirumab that is an anti-VEGFR-2 antibody, fam-trastuzumab deruxtecan-nxki, an antibody drug conjugate against HER2 and, single or combination chemotherapy are recommended.

Since the conventional anticancer agent aims to kill cancer cells by cell injury while a molecular target drug, such as an antibody drug, targets molecules excessively expressing in cancer cells, the action mechanisms thereof differ from each other. As a result, there is a high likelihood that the effect can also be obtained for patients who have not obtained the effect. Also, for a molecular target therapeutic drug against stomach cancer, a new antibody medicine has been developed.

However, only the agents that target limited growth factors such as HER2, VEGF, or the like can be currently used against advanced stomach cancer. The number of cases of HER2 positive stomach cancer is around 20% of that of the advanced and recurrence stomach cancers, and trastuzumab is effective only to the HER2 positive stomach cancers. Further, the response rate of nivolumab that is a newly approved immune checkpoint inhibitor also still remains at around 20%, which results in limitation of treatment. Further, ramucirumab approved in the treatment for advanced stomach cancers in the United States does not have so high a prolonging effect of prognostics. Current median life expectancy of patients with metastatic gastric cancer relapsing after initial treatment is between 6-10 months. Thus, development of an inhibitor that may control progression of a stomach cancer from a completely different action mechanism is essential for improving a treatment outcome.

Recently, the neuronal pentraxin receptor NPTXR has been disclosed as promising target for cancer therapy. In EP 3 744 346 A1, a particular peptide of NPTXR has been identified which allows for generation of antibodies which are suitable for diagnosis and/or therapy of cancer including stomach cancer, colorectal cancer, liver cancer, bladder cancer, breast cancer, esophageal cancer, glioblastoma, glioma, lung cancer, malignant melanoma, ovarian cancer, malignant pheochromocytoma, prostatic cancer, testicular germ cell tumor, thyroid cancer, pancreatic cancer, uterine cancer, and renal cell cancer. Further, EP 3 744 346 A1 discloses a corresponding antibody derived from a hybridoma represented by accession no. NITE P-02856 which specifically binds to said specific peptide of NPTXR consisting of an amino acid sequence of GLPRGLQGAGPRRDT (SEQ ID NO: 2) or CESGLPRGLQGAGPRRDT (SEQ ID NO: 3).

The object of the present invention is to provide further antibodies binding specifically to NPTXR which have improved properties compared to the known antibody binding to the same epitope.

### DESCRIPTION OF THE INVENTION:

The present invention is directed to an isolated antibody or antigen binding fragment thereof as defined in the claims that binds specifically to human NPTXR with the amino acid sequence of SQ ID NO: 1; preferably, the antibody or antigen binding fragment of the present invention specifically binds to a peptide consisting of an amino acid sequence of CESGLPRGLQDAGPRRDT (SEQ ID NO: 14), or of GLPRGLQGAGPRRDT (SEQ ID NO: 2), or of CESGLPRGLQGAGPRRDT (SEQ ID NO: 3).

Preferably, the isolated antibody or antigen binding fragment of the invention comprises three heavy chain complementarity determining regions (HCDR1, HCDR2, and HCDR3), and three light chain complementarity determining regions (LCDR1, LCDR2, and LCDR3), wherein:
i) HCDR1 comprises or consists of the amino acid sequence of NYGMN (SEQ ID NO: 4);
ii) HCDR2 comprises or consists of the amino acid sequence of WINTDIGEPTYSQKFQG (SEQ ID NO: 5); and
iii) HCDR3 comprises or consists of the amino acid sequence of EMDV (SEQ ID NO: 6); and wherein
iv) LCDR1 comprises or consists of the amino acid sequence of RASQSISDYLH (SEQ ID NO: 7);
v) LCDR2 comprises or consists of the amino acid sequence of YVSHSIS (SEQ ID NO: 8);
   and
vi) LCDR3 comprises or consists of the amino acid sequence of LNGHSFPFT (SEQ ID NO: 9).

The inventors have identified a novel antibody that specifically binds to human NPTXR, in particular to the N-domain of the extracellular part of human NPTXR represented by a peptide consisting of an amino acid sequence of GLPRGLQGAGPRRDT (SEQ ID NO: 2), or of CESGLPRGLQGAGPRRDT (SEQ ID NO: 3), while said antibody shows improved properties compared to antibodies of the prior art. The antibody of the invention shows a significantly improved EC50 value for human NPTXR compared to known antibodies against the same epitope of human NPTXR. The parameter EC50 abbreviates for 'half maximal effective concentration', and, in a pharmacological context, this EC50 parameter correlates to the concentration of a drug that is necessary to cause half of the maximum possible effect. Thus, a lower EC50 value may allow for administration of a lower dose while achieving the same clinical effect.

It has surprisingly been found that the antibody of the invention exhibits significantly improved EC50 values not only for human NPTXR but also for mouse NPTXR represented by a peptide consisting of an amino acid sequence of CESGLPRGLQDAGPRRDT (SEQ ID NO: 14). Thus, the antibody of the invention is particularly suitable not only for diagnosis and therapy of certain types of cancer in human subjects but provides also an improved research tool which shows comparable EC50 values for human NPTXR and mouse NPTXR.

Further, the antibody of the invention showed improved antibody characteristics in an analytical HPLC-SEC. The antibody of the invention behaves extremely well under stress condition such as high temperature stress and stress induced by low pH. After such temperature stress the monomeric peak is nearly identical to the control sample without stress induction. Stress induction using a low pH (pH 3.0) for 24 hours resulted also in no aggregation or degradation of the antibody.

The isolated antibody or antigen binding fragment thereof of the invention preferably comprises a heavy chain (VH) comprising or consisting of the amino acid sequence of: 
QVQLVQSGAEVKKPGASVKVSCKASGYTFTNYGMNWVRQAPGQRLEWMGWINTDIGEPT
YSQKFQGRVTITRDTSASTAYMELSSLRSEDTAVYYCAREMDVWGQGTTVTVSS

(SEQ ID NO: 10),
 and a light chain (VL) comprising or consisting of the amino acid sequence of:

DIQMTQSPSSLSASVGDRVTITCRASQSISDYLHWYQQKPGKAPKLLIKYVSHSISGVPSRF
SGSGSGTDFTLTISSLQPEDFATYYCLNGHSFPFTFGGGTKVEIK

(SEQ ID NO: 11).

In a preferred embodiment, the VH region with the amino acid sequence of SEQ ID NO: 10 is encoded by a nucleic acid molecule comprising the nucleotide sequence of:

CAGGTGCAGCTGGTGCAGTCTGGCGCTGAGGTGAAGAAGCCTGGCGCCTCTGTGAAG
GTGTCCTGCAAGGCCTCCGGCTATACCTTCACCAACTACGGAATGAACTGGGTCAGACA
AGCCCCTGGACAGAGACTGGAGTGGATGGGGTGGATCAACACCGACATCGGGGAGCC
CACCTACTCCCAGAAGTTCCAGGGGCGGGTCACAATCACCCGGGATACCTCCGCAAGC
ACCGCTTACATGGAGCTGTCTTCTCTGCGCAGCGAGGACACCGCTGTCTACTACTGCG
CTCGCGAGATGGATGTCTGGGGACAGGGGACAACAGTCACCGTCAGCAGC

(SEQ ID NO: 12).

Alternatively or in addition, the VL region with the amino acid region of SEQ ID NO: 11 is encoded by a nucleic acid molecule comprising the nucleic acid sequence

of:GACATCCAGATGACCCAGTCTCCCTCTTCTCTGTCTGCCTCTGTGGGCGATCGGGTG
ACCATCACCTGTCGGGCTTCCCAGTCCATCTCCGACTACCTGCACTGGTACCAGCAGAA
GCCTGGCAAGGCCCCAAAGCTGCTCATCAAGTACGTGTCCCACTCCATCTCCGGCGTG
CCATCTAGATTCTCTGGATCTGGATCTGGAACCGACTTCACCCTCACCATCTCCTCCCT
CCAGCCTGAGGACTTCGCCACCTACTACTGCCTCAACGGGCACTCTTTCCCTTTCACCT
TCGGAGGAGGGACCAAGGTGGAGATCAAG

(SEQ ID NO: 13).

The isolated antibody or antigen binding fragment thereof of the invention is preferably monoclonal.

The isolated antibody or antigen binding fragment thereof of the invention preferably is:
- recombinant; or
- an IgG, IgM, IgA or an antigen binding fragment thereof; or
- a Fab fragment, a Fab' fragment, a F(ab')2 fragment, a F(ab')3 fragment, a Fd fragment, a Fd' fragment, a Fv fragment, a scFv, a bivalent scFv, a diabody, a linear antibody, or a monovalent.

The isolated antibody or antigen binding fragment thereof of the invention is preferably a humanized antibody or de-immunized antibody or an antigen binding fragment thereof.

The isolated antibody or antigen binding fragment thereof of the invention can be monospecific or bi-specific.

The isolated antibody or antigen binding fragment thereof of the invention optionally is conjugated to another molecule to form an immunoconjugate, preferably the isolated antibody or antigen binding fragment thereof of the invention is conjugated to an active agent like e.g. an imaging agent, a therapeutic agent, a toxin or a radionuclide.

The present invention is further directed to a pharmaceutical composition comprising the isolated antibody or antigen binding fragment thereof of the invention and a pharmaceutically or physiologically acceptable carrier or excipient.

Further, the present invention refers to a polynucleotide molecule comprising a nucleic acid sequence encoding an isolated antibody or antigen binding fragment thereof of the present invention.

The present invention is also directed to a host cell comprising one or more polynucleotide molecule(s) encoding an isolated antibody or antigen binding fragment thereof of the invention, optionally wherein the host cell is a mammalian cell, a yeast cell, a bacterial cell, a ciliate cell, an insect cell or a plant cell. The host cell can be a eukaryotic cell, e.g., a mammalian cell, an insect cell, a yeast cell, or a prokaryotic cell, e.g., E. coli. For example, the mammalian cell can be a cultured cell or a cell line. Exemplary mammalian cells include lymphocytic cell lines (e.g., NS0), Chinese hamster ovary cells (CHO), COS and HEK-293 cells. Additionally, cells include oocyte cells, and cells from a transgenic animal, e.g., mammary epithelial cell. For example, nucleic acids encoding an antibody or a modified antibody or an antigen binding fragment thereof described herein can be expressed in a transgenic animal.

Further, the present invention comprises a method of manufacturing an antibody or antigen binding fragment thereof of the invention comprising:
(a) expressing one or more polynucleotide molecule(s) encoding an isolated antibody or antigen binding fragment thereof of the invention in a cell; and
(b) purifying the antibody from the cell/cell supernatants.

The present invention also refers to kit comprising an isolated antibody or antigen binding fragment of any of the invention and instructions for use of the antibody, optionally further wherein the antibody of antigen binding fragment thereof of the invention is lyophilized.

Further, the present invention is directed to a medical use of the antibody or antigen binding fragment thereof of the invention. Preferably, the isolated antibody or antigen binding fragment of the invention or the pharmaceutical composition of the invention is intended for use in a diagnostic method of a disease or medical condition. Alternatively or in addition, the isolated antibody or antigen binding fragment of the invention or the pharmaceutical composition of the invention is provided for use in treating or preventing a disease or medical condition. In one embodiment, the antibody or antigen binding fragment of the invention or the pharmaceutical composition of the invention is used in the manufacture of a medicament for treatment or prevention of a disease or medical condition. In another embodiment, the present invention is directed to a method of treatment or prevention of a disease or medical condition, wherein a subject in need thereof is administered with an effective amount of the antibody or antigen binding fragment thereof of the invention or the pharmaceutical composition of the invention. Preferably, the disease or medical condition referred to above comprise or consist of stomach cancer, colorectal cancer, liver cancer, bladder cancer, breast cancer, esophageal cancer, glioblastoma, glioma, lung cancer, malignant melanoma, ovarian cancer, malignant pheochromocytoma, cervical cancer, prostatic cancer, testicular germ cell tumor, thyroid cancer, pancreatic cancer, uterine cancer, and renal cell cancer, in particular of liver cancer, bladder cancer, thyroid cancer, stomach cancer, colorectal cancer, breast cancer, lung cancer, pancreatic cancer, or oesophageal cancer.

Unless otherwise defined herein, scientific and technical terms used in connection with the present invention have the meanings that are commonly understood by those of ordinary skill in the art. Generally, nomenclature utilized in connection with, and techniques of, cell and tissue culture, molecular biology, and protein and oligo- or polynucleotide chemistry and hybridization described herein are those well-known and commonly used in the art. Standard techniques are used for recombinant DNA, oligonucleotide synthesis, and tissue culture and transformation and transfection (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art; see e.g., Sambrook et al. Molecular Cloning: A Laboratory Manual (3rd ed., Cold Spring Harbor Laboratory Press, Cold Spring Harbor, N.Y. (2000)), and Harlow, E. and Lane, D. (1988) Antibodies: A Laboratory Manual, Cold Spring Harbor Laboratory Press, Cold Spring Harbor, NY. The nomenclatures utilized in connection with, and the laboratory procedures and techniques of, analytical chemistry, synthetic organic chemistry, and medicinal and pharmaceutical chemistry described herein are those well-known and commonly used in the art. Standard techniques are used for chemical syntheses, chemical analyses, pharmaceutical preparation, formulation, and delivery, and treatment of patients. Furthermore, unless otherwise required by context, singular terms shall include pluralities and plural terms shall include the singular.

The instant invention is most clearly understood with reference to the following definitions, as well as additional definitions as set forth throughout the description:
As used herein, the term "antibody", "antibody peptide(s)" or "immunoglobulin" refers to single chain, two-chain, and multi-chain proteins and glycoproteins that belong to the classes of polyclonal, monoclonal, chimeric and human or humanized immunoglobulin proteins. The term "antibody" also includes synthetic and genetically engineered variants thereof.

As used herein, the term "antibody fragment" or "antigen binding fragment" of an antibody refers to a Fab fragment, a Fab' fragment, a F(ab')2 fragment, a F(ab')3 fragment, a Fd fragment, a Fd' fragment, a Fv fragment, a scFv, a bivalent scFv, a diabody, a linear antibody, single chain antibodies, functional heavy chain antibodies (nanobodies), as well as any portion of an antibody having specificity toward at least one desired epitope, that competes with the intact antibody for specific binding (e.g., an isolated portion of a complementarity determining region having sufficient framework sequences so as to bind specifically to an epitope). Antigen binding fragments can be produced by recombinant techniques, or by enzymatic or chemical cleavage of an intact antibody.

As used herein, the term "human antibody" refers to an antibody that possesses a sequence that is derived from a human germ-line immunoglobulin sequence, such as antibodies derived from transgenic mice having human immunoglobulin genes (e.g., XENOMOUSE ^{™}genetically engineered mice (Abgenix)), human phage display libraries, in cows (milk) or human B cells.

As used herein, the term "humanized antibody" refers to an antibody that is derived from a non-human antibody (e.g., murine) that retains or substantially retains the antigen-binding properties of the parent antibody but is less immunogenic in humans. Humanized as used herein is intended to include deimmunized antibodies.

The term "modified" or "recombinant" antibody, as used herein, refers to antibodies that are prepared, expressed, created or isolated by recombinant means, such as antibodies expressed using a recombinant expression vector transfected into a host cell, antibodies isolated from a recombinant, combinatorial antibody library, antibodies isolated from an animal (e.g., a mouse) that is transgenic for human immunoglobulin genes or antibodies prepared, expressed, created or isolated by any other means that involves splicing of human immunoglobulin gene sequences to other DNA sequences. Such modified antibodies include humanized, CDR grafted, chimeric, *in vitro* generated (e.g., by phage display) antibodies, and may optionally include variable or constant regions derived from human germline immunoglobulin sequences or human immunoglobulin genes or antibodies which have been prepared, expressed, created or isolated by any means that involves splicing of human immunoglobulin gene sequences to alternative immunoglobulin sequences.

The term "monospecific antibody" refers to an antibody that displays a single binding specificity and affinity for a particular target, e.g., epitope. This term includes a "monoclonal antibody" or "monoclonal antibody composition," which as used herein refer to a preparation of antibodies or fragments thereof of single molecular composition.

The term "bispecific antibody" or "bifunctional antibody" refers to an antibody that displays dual binding specificity for two epitopes, where each binding site differs and recognizes a different epitope.

It is understood that the antibodies and antigen binding fragment thereof of the invention may have additional conservative or non-essential amino acid substitutions, which do not have a substantial effect on the polypeptide functions. Whether or not a particular substitution will be tolerated, i.e., will not adversely affect desired biological properties, such as binding activity can be determined as described in Bowie, JU et al. Science 247:1306-1310 (1990). A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., asparagine, glutamine, serine, threonine, tyrosine, cysteine), nonpolar side chains (e.g., glycine, alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

A "non-essential" amino acid residue is a residue that can be altered from the wild-type sequence of the binding agent, e.g., the antibody, without abolishing or more preferably, without substantially altering a biological activity, whereas an "essential" amino acid residue results in such a change.

As used herein, the term "isolated" refers to material that is removed from its original environment (e.g., the natural environment if it is naturally occurring). For example, a naturally occurring polynucleotide or polypeptide present in a living animal is not isolated, but the same polynucleotide or DNA or polypeptide, separated from some or all of the coexisting materials in the natural system, is isolated. Such polynucleotide could be part of a vector and/or such polynucleotide or polypeptide could be part of a composition, and still be isolated in that the vector or composition is not part of its natural environment.

As used herein, the term "vector" refers to a replicon in which another polynucleotide segment is attached, such as to bring about the replication and/or expression of the attached segment.

As used herein, the term "control sequence" refers to a polynucleotide sequence that is necessary to effect the expression of a coding sequence to which it is ligated. The nature of such control sequences differs depending upon the host organism. In prokaryotes, such control sequences generally include a promoter, a ribosomal binding site and terminators and, in some instances, enhancers. The term "control sequence" thus is intended to include at a minimum all components whose presence is necessary for expression, and also may include additional components whose presence is advantageous, for example, leader sequences.

As used herein, the term "purified product" refers to a preparation of the product which has been isolated from the cellular constituents with which the product is normally associated and from other types of cells that may be present in the sample of interest.

As used herein, the term "epitope" refers to any protein determinate capable of binding specifically to an antibody or T-cell receptors. Epitopic determinants usually consist of chemically active surface groupings of molecules such as amino acids or sugar side chains and usually have specific three-dimensional structural characteristics, as well as specific charge characteristics.

As used herein, "isotype" refers to the antibody class (e.g., IgM; IgA or IgG) that is encoded by heavy chain constant region genes.

The term "agent" or "active agent" is used herein to denote a radionuclide, a chemical compound, a mixture of chemical compounds, a biological macromolecule, or an extract made from biological materials. The term "agent" or "active agent" comprises e.g. imaging agents, therapeutic agents, toxins and radionuclides.

As used herein, the terms "label" or "imaging agent" refers to a detectable marker that can be incorporated, e.g., by incorporation of a radiolabelled amino acid or attachment to a polypeptide of biotinyl moieties that can be detected by marked avidin (e.g., streptavidin containing a fluorescent marker or enzymatic activity that can be detected by optical or calorimetric methods). Useful detectable agents or imaging agents with which an antibody or an antibody portion of the invention may be labelled include fluorescent, infrared, x-ray, ultrasound compounds or coupled to e.g. air or gas filled material, metal and metal ligations (e.g. for photothermal therapy), various enzymes, prosthetic groups, luminescent materials, bioluminescent materials, fluorescent emitting metal atoms, e.g., europium (Eu), and other Lanthanides, and radioactive materials (described above). Exemplary fluorescent detectable agents include fluorescein, fluorescein isothiocyanate, rhodamine, 5-dimethylamine-1-napthalenesulfonyl chloride, phycoerythrin and the like. An antibody may also be derivatized with detectable enzymes, such as alkaline phosphatase, horseradish peroxidase, b-galactosidase, acetylcholinesterase, glucose oxidase and the like. When an antibody is derivatized with a detectable enzyme, it is detected by adding additional reagents that the enzyme uses to produce a detectable reaction product. For example, when the detectable agent horseradish peroxidase is present, the addition of hydrogen peroxide and diaminobenzidine leads to a coloured reaction product, which is detectable. An antibody may also be derivatized with a prosthetic group (e.g., streptavidin/biotin and avidin/biotin). For example, an antibody may be derivatized with biotin, and detected through indirect measurement of avidin or streptavidin binding. Examples of suitable fluorescent materials include umbelliferon, fluorescein, fluorescein isothiocyanate, rhodamine, dichlorotriazinylamine fluorescein, dansyl chloride or phycoerythrin; an example of a luminescent material includes luminol; and examples of bioluminescent materials include luciferase, luciferin, and aequorin. In certain situations, the label or imaging agent can also be therapeutic. Various methods of labelling polypeptides and glycoproteins are known in the art and may be used. Examples of labels or imaging agents for polypeptides like e.g. the antibody or the antigen binding fragment thereof of the invention include, but are not limited to, the following: radioisotopes or radionuclides (e.g., ³H, ¹⁴C, ¹⁵N, ³⁵S, ⁹⁰Y, ⁹⁹Tc, ¹¹¹In, ¹²⁵I, ¹³¹I), fluorescent labels (e.g., FITC, rhodamine, lanthanide phosphors), enzymatic labels (e.g., horseradish peroxidase, beta-galactosidase, luciferase, alkaline phosphatase), chemiluminescent, biotinyl groups, predetermined polypeptide epitopes recognized by a secondary reporter (e.g., leucine zipper pair sequences, binding sites for secondary antibodies, metal binding domains, epitope tags), metal and metal ligations. In some embodiments, labels or imaging agents are attached by spacer arms of various lengths to reduce potential steric hindrance.

Alternatively or in addition, the antibody or antigen binding fragment thereof of the invention can be conjugated to a therapeutic agent. Examples of therapeutic agents include, but are not limited to, cytochalasin B, gramicidin D, ethidium bromide, emetine, etoposide, tenoposide, colchicin, dihydroxy anthracin dione, mitoxantrone, 1-dehydrotestosterone, glucocorticoids, procaine, tetracaine, lidocaine, propranolol, antimetabolites (e.g., methotrexate, 6-mercaptopurine, 6-thioguanine, cytarabine, 5-fluorouracil decarbazine), alkylating agents (e.g., mechlorethamine, thioepa chlorambucil, CC-1065(see US Patent Nos. 5,475,092 , 5,585,499, 5,846,545 ), melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, mitomycin, puromycin anthramycin (AMC)), duocarmycin and analogs or derivatives thereof, and anti-mitotic agents (e.g., vincristine, vinblastine, taxol, auristatins (e.g., auristatin E) and maytansinoids, and topoisomerase inhibitors and analogs or homologs thereof.

Alternatively or in addition, the antibody of the invention can also be presented as bi-specific antibody, wherein said bi-specific antibody comprises an additional binding moiety which preferably is specific for an antigen which differs from NPTXR. Such a bi-specific antibody may comprise a binding motif specific for Her2, PD-1, Claudin 18.1 and/or CD3.

Alternatively or in addition, the antibody or antigen binding fragment thereof of the invention can also be coupled a radioactive isotope or radionuclide. Radioactive isotopes can be used in diagnostic or therapeutic applications. Radioactive isotopes that can be coupled to the antibodies include, but are not limited to α-, β-, or γ-emitters, or β- and γ-emitters. Such radioactive isotopes include, but are not limited to iodine (¹³¹I or ¹²⁵I), yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), indium (¹¹¹In), technetium (^{99m}Tc), phosphorus (³²P), rhodium (⁸⁸Rh), sulphur (³⁵S), carbon (¹⁴C), tritium (³H), chromium (⁵¹Cr), chlorine (³⁶Cl), cobalt (⁵⁷Co or ⁵⁸Co), iron (⁵⁹Fe), selenium (⁷⁵Se), or gallium (⁶⁷Ga). Radioisotopes useful as therapeutic agents include yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), and rhodium (¹⁸⁸Rh). Radioisotopes useful as labels, e.g., for use in diagnostics, include iodine (¹³¹I or ¹²⁵I), indium (¹¹¹In), technetium (^{99m}Tc), phosphorus (³²P), carbon (¹⁴C), and tritium (³H), or one or more of the therapeutic isotopes listed above.

As used herein, "specific binding" "bind(s) specifically" or "binding specificity" refers to the property of the antibody to bind to human NPTXR with an affinity of at least 10⁻⁷ M, preferably of at least 10⁻⁹ M.

As used herein, the term "treat" or "treatment" is defined as the application or administration of an antibody or antigen binding fragment thereof of the invention to a subject, e.g., a subject in need thereof or a patient, or application or administration to an isolated tissue or cell from a subject, e.g., a patient, which is returned to the subject. The antibody or antigen binding fragment thereof of the invention can be administered alone or in combination with a second agent. The treatment can be to cure, heal, alleviate, relieve, alter, remedy, ameliorate, palliate, improve or affect the disorder, disease or medical condition, the symptoms of the disorder or the predisposition toward the disorder.

Disorders or medical conditions that can be treated with the antibody or antigen binding fragment thereof of the invention comprise cancer, preferably stomach cancer, colorectal cancer, liver cancer, bladder cancer, breast cancer, esophageal cancer, glioblastoma, glioma, lung cancer, malignant melanoma, ovarian cancer, malignant pheochromocytoma, prostatic cancer, testicular germ cell tumor, thyroid cancer, pancreatic cancer, uterine cancer, and renal cell cancer, more preferably liver cancer, bladder cancer, thyroid cancer, stomach cancer, colorectal cancer, breast cancer, lung cancer, pancreatic cancer, and/or oesophageal cancer, even more preferably stomach cancer, colorectal cancer, breast cancer, lung cancer, pancreatic cancer, and/or oesophageal cancer.

As used herein, an amount of an antibody or antigen binding fragment thereof of the invention "effective" or "sufficient" to treat a disorder, or a "therapeutically effective amount" or "therapeutically sufficient amount "refers to an amount of the antibody or antigen binding fragment thereof of the invention which is effective, upon single or multiple dose administration to a subject, in treating a cell, or in prolonging, curing, alleviating, relieving or improving a subject with a disorder as described herein beyond that expected in the absence of such treatment.

As used herein, "human NPTXR" refers to human NPTXR with the amino acid sequence of:

MKFLAVLLAAGMLAFLGAVICIIASVPLAASPARALPGGADNASVASGAAASPGPQRSLSALH
GAGGSAGPPALPGAPAASAHPLPPGPLFSRFLCTPLAAACPSGAQQGDAAGAAPGEREEL
LLLQSTAEQLRQTALQQEARIRADQDTIRELTGKLGRCESGLPRGLQGAGPRRDTMADGP
WDSPALILELEDAVRALRDRIDRLEQELPARVNLSAAPAPVSAVPTGLHSKMDQLEGQLLAQ
VLALEKERVALSHSSRRQRQEVEKELDVLQGRVAELEHGSSAYSPPDAFKISIPIRNNYMYA
RVRKALPELYAFTACMWLRSRSSGTGQGTPFSYSVPGQANEIVLLEAGHEPMELLINDKVA
QLPLSLKDNGWHHICIAWTTRDGLWSAYQDGELQGSGENLAAWHPIKPHGILILGQEQDTL

GGRFDATQAFVGDIAQFNLWDHALTPAQVLGIANCTAPLLGNVLPWEDKLVEAFGGATKAA
FDVCKGRAKA

(SEQ ID NO: 1).

The human NPTXR protein comprises 500 amino acids, and is described in NCBI accession no.: NP_055108.2.

The antibodies or antigen binding fragments thereof of the invention interact with, e.g., bind to the extracellular N domain of human NPTXR. In one embodiment, the antibody or antigen binding fragment thereof of the invention specifically binds to a peptide consisting of an amino acid sequence of CESGLPRGLQDAGPRRDT (SEQ ID NO: 14), or of GLPRGLQGAGPRRDT (SEQ ID NO: 2), or of CESGLPRGLQGAGPRRDT (SEQ ID NO: 3).

### Antibodies:

The antibody structural unit is a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 120 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989)). The variable regions of each light chain (VL) and heavy chain (VH) pair form the antibody binding site. Preferred isotypes for the antibodies of the invention are IgG immunoglobulins, which are classified into four subclasses, IgG1, IgG2, IgG3 and IgG4, having different gamma heavy chains. Most therapeutic antibodies are human, chimeric, or humanized antibodies of the IgG1 type.

The variable regions of each heavy and light chain pair form the antigen binding site. Thus, an intact IgG antibody has two binding sites which are the same. However, bifunctional or bispecific antibodies are artificial hybrid constructs which have two different heavy/light chain pairs, resulting in two different binding sites.

The chains all exhibit the same general structure of relatively conserved framework regions (FR) joined by three hyper variable regions, also called complementarity determining regions or CDRs. The CDRs from the two chains of each pair are aligned by the framework regions, enabling binding to a specific epitope. From N-terminal to C-terminal, both light and heavy chains comprise the domains FR1, CDR1, FR2, CDR2, FR3, CDR3 and FR4. The assignment of amino acids to each domain is in accordance with the definitions of the IMTG unique Lefranc numbering, Lefranc, M.P., The Immunologist, 7,132-136(1999), homepage IMTG^{®} http://www.imtg.org. As used herein, CDRs are referred to for each of the heavy (HCDR1, HCDR2, HCDR3) and light (LCDR1, LCDR2, LCDR3) chains.

The antibodies of the present invention can be polyclonal antibodies, monoclonal antibodies, chimeric antibodies (see U.S. Pat. No. 6,020,153) or human or humanized antibodies or antibody fragments or derivatives thereof. Synthetic and genetically engineered variants (see U.S. Pat. No. 6,331,415) of any of the foregoing are also contemplated by the present invention. Monoclonal antibodies can be produced by a variety of techniques, including conventional murine monoclonal antibody methodology e.g., the standard somatic cell hybridization technique of Kohler and Milstein, Nature 256: 495 (1975). See generally, Harlow, E. and Lane, D. (1988) Antibodies: A Laboratory Manual, Cold Spring Harbor Laboratory Press, Cold Spring Harbor, NY. Preferably, the antibodies of the present invention are human or humanized antibodies. The advantage of human or humanized antibodies is that they potentially decrease or eliminate the immunogenicity of the antibody in a host recipient, thereby permitting an increase in the bioavailability and a reduction in the possibility of adverse immune reaction, thus potentially enabling multiple antibody administrations.

Modified antibodies include humanized, chimeric or CDR-grafted antibodies. Human antimouse antibody (HAMA) responses have led to development of chimeric or otherwise humanized antibodies. While chimeric antibodies have a human constant region and a murine variable region, it is expected that certain human anti-chimeric antibody (HACA) responses will be observed, particularly in chronic or multi-dose utilizations of the antibody. The presence of such murine or rat derived proteins can lead to the rapid clearance of the antibodies or can lead to the generation of an immune response against the antibody by a patient. In order to avoid the utilization of murine or rat derived antibodies, humanized antibodies where sequences are introduced to an antibody sequence to make it closer to human antibody sequence, or fully human antibodies generated by the introduction of human antibody function into a rodent have been developed so that the rodent would produce antibodies having fully human sequences. Human antibodies avoid certain of the problems associated with antibodies that possess murine, rabbit, or rat variable and/or constant regions.

### Human Antibodies:

Fully human antibodies are expected to minimize the immunogenic and allergic responses intrinsic to mouse or mouse-derivatized mAbs and thus to increase the efficacy and safety of the administered antibodies. The use of fully human antibodies can be expected to provide a substantial advantage in the treatment of chronic and recurring human diseases, such as inflammation, autoimmunity, and cancer, which require repeated antibody administrations. Also, human antibodies can be produced using genetically engineered strains of animals in which the antibody gene expression of the animal is suppressed and functionally replaced with human antibody gene expression.

Methods for making humanized and human antibodies are known in the art. One method for making human antibodies employs the use of transgenic animals, such as a transgenic mouse. These transgenic animals contain a substantial portion of the human antibody producing genome inserted into their own genome and the animal's own endogenous antibody production is rendered deficient in the production of antibodies. Methods for making such transgenic animals are known in the art. Such transgenic animals can be made using XENOMOUSE^{™} technology or by using a "minilocus" approach. Methods for making XENOMICE^{™} are described in U.S. Pat. Nos. 6,162,963, 6,150,584, 6,114,598 and 6,075,181. Methods for making transgenic animals using the "minilocus" approach are described in U.S. Pat. Nos. 5,545,807, 5,545,806 and 5,625,825; also see International Publication No. WO93/12227.

Using the XENOMOUSE^{™} technology, human antibodies can be obtained by immunizing a XENOMOUSE^{™} mouse (Abgenix, Fremont, Calif.) with an antigen of interest. The lymphatic cells (such as B-cells) are recovered from the mice that express antibodies. These recovered cells can be fused with myeloid-type cell line to prepare immortal hybridoma cell lines, using standard methodology. These hybridoma cell lines can be screened and selected to identify hybridoma cell lines that produce antibodies specific to the antigen of interest. Alternatively, the antibodies can be expressed in cell lines other than hybridoma cell lines. More specifically, sequences encoding particular antibodies can be cloned from cells producing the antibodies and used for transformation of a suitable mammalian host cell. In a preferred method, spleen and/or lymph node lymphocytes from immunized mice are isolated from the mice and plated in plaque assays as known in the art. Briefly, cells are plated in agar with sheep red blood cells, coated with antigen and cells secreting mAb against the antigen would fix complement and lyse the red blood cells immediately surrounding the mAb producing cells. Cells within the cleared plaques are lifted for sequencing of the immunoglobulin sequences and subcloning into expression vectors. Supernatants from transiently transfected cells containing antigen-specific mAb were subsequently screened by ELISA and for binding to cells by flow cytometry. The variable sequences, or a portion thereof of the produced human antibodies comprising complementarity determining regions which bind particular epitopes may be utilized for production of modified antibodies. For example, the variable regions of the produced antibodies may be spliced into an expression cassette for ease of transfer of constructs, increased expression of constructs, and/or incorporation of constructs into vectors capable of expression of full-length antibodies.

### Chimerization, Humanization and Display Technologies:

As discussed above, there are advantages to producing antibodies with reduced immunogenicity. To a degree, this can be accomplished in connection with techniques of humanization and display techniques using appropriate libraries. It will be appreciated that murine antibodies or antibodies from other species can be humanized or primatized using techniques well known in the art. The antibody of interest may be engineered by recombinant DNA techniques to substitute the CH1, CH2, CH3, hinge domains, and/or the framework domain with the corresponding human sequence (see e.g. WO 92/02190 A1, US 5,530,101, US 5,585,089, US 5,693,761, US 5,693,792, US 5,714,350, and US 5,777,085). Also, the use of Ig cDNA for construction of chimeric immunoglobulin genes is known in the art. mRNA is isolated from a hybridoma or other cell producing the antibody and used to produce cDNA: The cDNA of interest may be amplified by the polymerase chain reaction using specific primers (see e.g. US 4,683,195 and US 4,683,202). Alternatively, a library is made and screened to isolate the sequence of interest. The DNA sequence encoding the variable region of the antibody is then fused to human constant region sequences. The sequences of human constant regions genes may be found in Kabat et al. (1991) Sequences of Proteins of Immunological Interest, N.I.H. publication no. 91-3242. Human C region genes are readily available from known clones. The choice of isotype will be guided by the desired effector functions, such as complement fixation, or activity in antibody-dependent cellular cytotoxicity. Isotypes can be IgG1, IgG2, IgG3 or IgG4. Preferred isotypes for antibodies of the invention are IgG1 and IgG2. Either of the human light chain constant regions, kappa or lambda, may be used. The chimeric, humanized antibody is then expressed by conventional methods.

Humanized antibodies can also be made using a CDR-grafted approach. Techniques of generation of such humanized antibodies are well known in the art. Generally, humanized antibodies are produced by obtaining nucleic acid sequences that encode the variable heavy and variable light sequences of an antibody that binds to the antigen of interest, identifying the complementary determining region or "CDR" in the variable heavy and variable light sequences and grafting the CDR nucleic acid sequences on to human framework nucleic acid sequences (see, for example, US 4,816,567 and US 5,225,539). The location of the CDRs and framework residues can be determined using known technology. The human framework that is selected is one that is suitable for *in vivo* administration, meaning that it does not exhibit immunogenicity. For example, such a determination can be made by prior experience with *in vivo* usage of such antibodies and studies of amino acid similarities.

Once the CDRs and FRs of the cloned antibody that are to be humanized are identified, the amino acid sequences encoding the CDRs are identified and the corresponding nucleic acid sequences grafted on to selected human FRs. This can be done using known primers and linkers, the selection of which are known in the art. Alternatively, the entire variable regions can be chemically synthesized without the need of a template. All of the CDRs of a particular human antibody may be replaced with at least a portion of a non-human CDR or only some of the CDRs may be replaced with non-human CDRs. It is only necessary to replace the number of CDRs required for binding of the humanized antibody to a predetermined antigen. After the CDRs are grafted onto selected human FRs, the resulting "humanized" variable heavy and variable light sequences are expressed to produce a humanized Fv or humanized antibody that binds to the antigen of interest. Typically, the humanized variable heavy and light sequences are expressed as a fusion protein with human constant domain sequences so an intact antibody that binds to the antigen of interest is obtained. However, a humanized Fv antibody can be produced that does not contain the constant sequences.

Also within the scope of the invention are humanized antibodies in which specific amino acids have been substituted, deleted or added. In particular, humanized antibodies have amino acid substitutions in the framework region, such as to improve binding to the antigen. For example, a selected, small number of acceptor framework residues of the humanized immunoglobulin chain can be replaced by the corresponding donor amino acids. Locations of the substitutions include amino acid residues adjacent to the CDR, or which are capable of interacting with a CDR (see e.g. US 5,585,089). The acceptor framework can be a mature human antibody framework sequence or a consensus sequence. As used herein, the term "consensus sequence" refers to the sequence formed from the most frequently in a region among related family members.

Other techniques for humanizing antibodies are described in EP 519596 A1. In addition, the invention covers also the humanization by guided selection and chain shuffling, where the murine variable domains are sequentially and functionally replaced by complementary human region displayed on filamentous phages.

The antibody or antigen binding fragment thereof of the invention includes other humanized antibodies which may also be modified by specific deletion of human T cell epitopes or "deimmunization" by the methods disclosed in WO 98/52976 A1 and WO 00/34317 A1. Briefly, the murine heavy and light chain variable regions of an antibody can be analysed for peptides that bind to MHC Class II; these peptides represent potential T-cell epitopes. For detection of potential T-cell epitopes, a computer modelling approach termed "peptide threading" can be applied, and in addition a database of human MHC class II binding peptides can be searched for motifs present in the murine VH and VL sequences, as described in WO 98/52976 A1 and WO 00/34317 A1. These motifs bind to any of the 18 major MHC class II DR allotypes, and thus constitute potential T cell epitopes. Potential T-cell epitopes detected can be eliminated by substituting small numbers of amino acid residues in the variable regions, or preferably, by single amino acid substitutions. As far as possible conservative substitutions are made, often but not exclusively, an amino acid common at this position in human germline antibody sequences may be used. The V BASE directory provides a comprehensive directory of human immunoglobulin variable region sequences (compiled by Tomlinson, I.A. et al. MRC Centre for Protein Engineering, Cambridge, UK). After the deimmunized VH and VL of an antibody are constructed by mutagenesis of the murine VH and VL genes, the mutagenized variable sequence can, optionally, be fused to a human constant region, e.g., human IgG1 or κ constant regions.

Antibodies of the invention that are not intact antibodies are also useful in this invention. Such antibodies may be derived from any of the antibodies described above. For example, antigen-binding fragments, as well as full-length monomeric, dimeric or trimeric polypeptides derived from the above-described antibodies are themselves useful. Useful antibody homologs of this type include (i) a Fab fragment, a monovalent fragment consisting of the VL, VH, CL and CH1 domains; (ii) a F(ab')2 fragment, a bivalent fragment comprising two Fab fragments linked by a disulphide bridge at the hinge region; (iii) a Fd fragment consisting of the VH and CH1 domains; (iv) a Fv fragment consisting of the VL and VH domains of a single arm of an antibody, (v) a dAb fragment, which consists of a VH domain; (vii) a single domain functional heavy chain antibody, which consists of a VHH domain (known as a nanobody).

Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules, known as single chain Fv (scFv). Such single chain antibodies are also intended to be encompassed within the term "antigen-binding fragment" of an antibody. These antibody fragments are obtained using conventional techniques known to those with skill in the art, and the fragments are screened for utility in the same manner as are intact antibodies. Antibody fragments, such as Fv, F(ab')2 and Fab may be prepared by cleavage of the intact protein, e.g. by protease or chemical cleavage.

In one approach, consensus sequences encoding the heavy and light chain J regions may be used to design oligonucleotides for use as primers to introduce useful restriction sites into the J region for subsequent linkage of V region segments to human C region segments. C region cDNA can be modified by site directed mutagenesis to place a restriction site at the analogous position in the human sequence.

Expression vectors include plasmids, retroviruses, cosmids, YACs, EBV derived episomes, and the like. A convenient vector is one that encodes a functionally complete human CH or CL immunoglobulin sequence, with appropriate restriction sites engineered so that any VH or VL sequence can be easily inserted and expressed. In such vectors, splicing usually occurs between the splice donor site in the inserted J region and the splice acceptor site preceding the human C region, and also at the splice regions that occur within the human CH exons. Polyadenylation and transcription termination occur at native chromosomal sites downstream of the coding regions. The resulting chimeric antibody may be joined to any strong promoter, Examples of suitable vectors that can be used include those that are suitable for mammalian hosts and based on viral replication systems, such as simian virus 40 (SV40), Rous sarcoma virus (RSV), adenovirus 2, bovine papilloma virus (BPV), papovavirus BK mutant (BKV), or mouse and human cytomegalovirus (CMV), and moloney murine leukaemia virus (MMLV), native Ig promoters, etc.

Expression in eukaryotic host cells is useful because such cells are more likely than prokaryotic cells to assemble and secrete a properly folded and immunologically active antibody. However, any antibody produced that is inactive due to improper folding may be re-naturable according to well-known methods. It is possible that the host cells will produce portions of intact antibodies, such as light chain dimers or heavy chain dimers, which also are antibody homologs according to the present invention.

Further, human antibodies or antibodies from other species can be generated through display-type technologies, including, without limitation, phage display, retroviral display, ribosomal display, and other techniques, using techniques well known in the art and the resulting molecules can be subjected to additional maturation, such as affinity maturation, as such techniques are well known in the art. If display technologies are utilized to produce antibodies that are not human, such antibodies can be humanized as described above.

It will be appreciated that antibodies that are generated need not initially possess a particular desired isotype but, rather, the antibody as generated can possess any isotype and the antibody can be isotype switched thereafter using conventional techniques that are well known in the art. Such techniques include the use of direct recombinant techniques (see e.g. US 4,816,397), cell-cell fusion techniques (see e.g. US 5,916,771), among others. In the cell-cell fusion technique, a myeloma or other cell line is prepared that possesses a heavy chain with any desired isotype and another myeloma or other cell line is prepared that possesses the light chain. Such cells can, thereafter, be fused and a cell line expressing an intact antibody can be isolated.

In connection with bispecific antibodies, bispecific antibodies can be generated that comprise (i) two antibodies one with a specificity to human NPTXR and another to a second molecule that are conjugated together, (ii) a single antibody that has one chain specific to human NPTXR and a second chain specific to a second molecule, or (iii) a single chain antibody that has specificity to human NPTXR and the other molecule. Such bispecific antibodies can be generated using techniques that are well known. For example, bispecific antibodies may be produced by crosslinking two or more antibodies (of the same type or of different types). Suitable crosslinkers include those that are heterobifunctional, having two distinctly reactive groups separated by an appropriate spacer (e.g., m-maleimidobenzoyl-N-hydroxysuccinimide ester) or homobifunctional (e.g., disuccinimidyl suberate).

### Nucleic acid and Polypeptides:

In another embodiment, the present invention relates to polynucleotide and polypeptide sequences that encode for the antibodies or fragments thereof described herein. Such polynucleotides encode for both the variable and constant regions of each of the heavy and light chains, although other combinations are also contemplated by the present invention in accordance with the compositions described herein. The present invention also contemplates oligonucleotide fragments derived from the disclosed polynucleotides and nucleic acid sequences complementary to these polynucleotides.

The polynucleotides can be in the form of RNA or DNA. Polynucleotides in the form of DNA, cDNA, genomic DNA, nucleic acid analogues and synthetic DNA are within the scope of the present invention. The DNA may be double-stranded or single-stranded, and if single stranded, may be the coding (sense) strand or non-coding (anti-sense) strand. The coding sequence that encodes the polypeptide may be identical to the coding sequence provided herein or may be a different coding sequence which coding sequence, as a result of the redundancy or degeneracy of the genetic code, encodes the same polypeptide as the DNA provided herein.

The provided polynucleotides encode at least one heavy chain variable region and at least one light chain variable region of the present invention. Examples of such polynucleotides are shown in SEQ ID NOS: 12 and 13 as well as fragments, complements and degenerate codon equivalents thereof. For example, the polynucleotide with SEQ ID NO: 12 encodes for the heavy chain variable region VH with SEQ ID NO: 10 comprising HCDR1 with SEQ ID NO: 4, HCDR2 with SEQ ID NO: 5 and HCDR3 with SEQ ID NO: 6; and the polynucleotide with SEQ ID NO: 13 encodes for the light chain variable region VL with SEQ ID NO: 11 comprising LCDR1 with SEQ ID NO: 7, LCDR2 with SEQ ID NO: 8 and LCDR3 with SEQ ID NO: 9.

The present invention also includes variant polynucleotides containing modifications such as polynucleotide deletions, substitutions or additions, and any polypeptide modification resulting from the variant polynucleotide sequence. A polynucleotide of the present invention may also have a coding sequence that is a variant of the coding sequence provided herein.

The present invention further discloses polypeptides that comprise or form part of the antibodies or antigen binding fragment thereof of the present invention as well as fragments, analogues and derivatives of such polypeptides. The polypeptides may be recombinant polypeptides, naturally produced polypeptides or synthetic polypeptides. The fragment, derivative or analogues of the polypeptides may be one in which one or more of the amino acid residues is substituted with a conserved or non-conserved amino acid residue (preferably a conserved amino acid residue) and such substituted amino acid residue may or may not be one encoded by the genetic code; or it may be one in which one or more of the amino acid residues includes a substituent group; or it may be one in which the polypeptide is fused with another compound, such as a compound to increase the half-life of the polypeptide (for example, polyethylene glycol); or it may be one in which the additional amino acids are fused to the polypeptide, such as a leader or secretory sequence or a sequence that is employed for purification of the polypeptide or a proprotein sequence. In various aspects, the polypeptides may be partially purified.

A polypeptide may have an amino acid sequence that is identical to that of the antibodies or antigen binding fragment thereof described herein or that is different by minor variations due to one or more amino acid substitutions. The variation may be a "conservative change" typically in the range of about 1 to 5 amino acids, wherein the substituted amino acid has similar structural or chemical properties, e.g., replacement of leucine with isoleucine or threonine with serine; replacement of lysine with arginine or histidine. In contrast, variations may include non-conservative changes, e.g., replacement of a glycine with a tryptophan. Similar minor variations may also include amino acid deletions or insertions or both. Guidance in determining which and how many amino acid residues may be substituted, inserted, or deleted without changing biological or immunological activity may be found using computer programs well known in the art, for example DNASTAR software (DNASTAR, Inc., Madison, Wis.).

The provided polypeptides encode at least one heavy chain variable region or at least one light chain variable region of the antibody or antigen binding fragment thereof of the present invention. The provided polypeptides can encode at least one heavy chain variable region and one light chain variable region of the antibodies of the present invention. Examples of such polypeptides are those having the amino acid sequences shown in SEQ ID NOS: 4, 5, 6, 7, 8, 9, 10 and 11, and fragments thereof. Preferably, the heavy variable region VH has the amino acid sequence shown in SEQ ID NO: 10 and the light variable region VL has the amino acid sequence shown in SEQ ID NO: 11. The heavy chain CDR sequences of the antibody or antigen binding fragment thereof of the invention have the amino acid sequence shown in SEQ ID NO: 4 (HCDR1), SEQ ID NO: 5 (HCDR2) and SEQ ID NO: 6 (HCDR3); and the light chain CDRs have the amino acid sequence shown in SEQ ID NO: 7 (LCDR1); SEQ ID NO: 8 (LCDR2); and SEQ ID NO: 9 (LCDR3).

The present invention also provides vectors that include the polynucleotides of the present invention, host cells which are genetically engineered with vectors of the present invention and the production of the antibodies of the present invention by recombinant techniques.

The appropriate DNA sequence may be inserted into the vector by a variety of procedures. In general, the DNA sequence is inserted into appropriate restriction endonuclease sites by procedures known in the art. The polynucleotide sequence in the expression vector is operatively linked to an appropriate expression control sequence (i.e. promoter) to direct mRNA synthesis. Examples of such promoters include, but are not limited to, the LTR or the SV40 promoter, the E. coli lac or trp, the phage lambda PL promoter and other promoters known to control expression of genes in prokaryotic or eukaryotic cells or their viruses. The expression vector also contains a ribosome binding site for translation initiation and a transcription terminator. The vector may also include appropriate sequences for amplifying expression. For example, the vector can contain enhancers, which are transcription-stimulating DNA sequences of viral origin, such as those derived from simian virus such as SV40, polyoma virus, cytomegalovirus, bovine papilloma virus or Moloney sarcoma virus, or genomic, origin. The vector preferably also contains an origin of replication. The vector can be constructed to contain an exogenous origin of replication or, such an origin of replication can be derived from SV40 or another viral source, or by the host cell chromosomal replication mechanism.

In addition, the vectors optionally contain a marker gene for selection of transfected host cells such as dihydrofolate reductase or antibiotics, such as neomycin, G418 (geneticin, a neomycin-derivative) or hygromycin, or genes which complement a genetic lesion of the host cells such as the absence of thymidine kinase, hypoxanthine phosphoribosyl transferase, dihydrofolate reductase, glutamine synthetase etc.

In order to obtain the antibodies of the present invention, one or more polynucleotide sequences that encode for the light and heavy chain variable regions and light and heavy chain constant regions of the antibodies of the present invention should be incorporated into a vector. Polynucleotide sequences encoding the light and heavy chains of the antibodies of the present invention can be incorporated into one or multiple vectors and then incorporated into the host cells.

As will be appreciated, antibodies in accordance with the present invention can be expressed in cell lines other than hybridoma cell lines. Sequences encoding the cDNAs or genomic clones for the particular antibodies can be used for a suitable mammalian or non-mammalian host cells. Transformation can be by any known method for introducing polynucleotides into a host cell, including, for example packaging the polynucleotide in a virus (or into a viral vector) and transducing a host cell with the virus (or vector) or by transfection procedures known in the art, for introducing heterologous polynucleotides into mammalian cells, e.g., dextran-mediated transfection, calcium phosphate precipitation, polybrene mediated transfection, protoplast fusion, electroporation, encapsulation of the polynucleotide(s) into liposomes and direct microinjection of the DNA molecule. The transformation procedure used depends upon the host to be transformed. Methods for introduction of heterologous polynucleotides into mammalian cells are well known in the art and include, but are not limited to, dextran-mediated transfection, calcium phosphate precipitation, polybrene mediated transfection, protoplast fusion, electroporation, particle bombardment, encapsulation of the polynucleotide(s) in liposomes, peptide conjugates, dendrimers, and direct microinjection of the DNA into nuclei.

Mammalian cell lines available as hosts for expression are well known in the art and include many immortalized cell lines available from the American Type Culture Collection (ATCC), including but not limited to Chinese hamster ovary (CHO) cells, NSO cells, HeLa cells, baby hamster kidney (BHK) cells, monkey kidney cells (COS), human hepatocellular carcinoma cells (e.g., Hep G2), HEK-293 and a number of other cell lines. Non-mammalian cells including but not limited to bacterial, yeast, insect, and plants can also be used to express recombinant antibodies. Site directed mutagenesis of the antibody CH2 domain to eliminate glycosylation may be preferred in order to prevent changes in either the immunogenicity, pharmacokinetic, and/or effector functions resulting from non-human glycosylation. The expression methods are selected by determining which system generates the highest expression levels and produce antibodies with constitutive antigen binding properties.

Further, expression of antibodies of the invention (or other moieties therefrom) from production cell lines can be enhanced using a number of known techniques. For example, the glutamine synthetase and DHFR gene expression systems are common approaches for enhancing expression under certain conditions. High expressing cell clones can be identified using conventional techniques, such as limited dilution cloning, Microdrop technology, or any other methods known in the art. The GS system is discussed in whole or part in connection with European Patent Nos. EP 0 216 846, EP 0 256 055, EP 0 338 841 and EP 0 323 997.

In an exemplary system for recombinant expression of a modified antibody, or antigen-binding portion thereof, of the invention, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to enhancer/promoter regulatory elements (e.g., derived from SV40, CMV, adenovirus and the like, such as a CMV enhancer/AdMLP promoter regulatory element or an SV40 enhancer/AdMLP promoter regulatory element) to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

Antibodies of the invention can also be produced transgenically through the generation of a mammal or plant that is transgenic for the immunoglobulin heavy and light chain sequences of interest and production of the antibody in a recoverable form therefrom. In connection with the transgenic production in mammals, antibodies can be produced in, and recovered from, the milk of goats, cows, or other mammals; see, e.g., US 5,827,690, US 5,756,687, US 5,750,172, and US 5,741,957.

### Conjugates:

As used herein, "immunoconjugate" comprises an antibody or antigen binding fragment of the invention, which is conjugated to a moiety comprising an agent or active agent or modified as described in more detail herein.

As used herein, a "moiety" of an immunoconjugate is intended to refer to a component of the conjugate (e.g., an immunoglobulin moiety (i.e., an antibody or antigen binding fragment or derivative thereof), a therapeutic moiety, an imaging moiety). An antibody or an antigen binding fragment thereof of the invention may be conjugated to another molecular entity, e.g., a cytotoxic or cytostatic agent, e.g., a therapeutic agent, a drug, a compound emitting radiation, molecules of plant, fungal, or bacterial origin, or a biological protein (e.g., a protein toxin) or particle (e.g., a recombinant viral particle, e.g., via a viral coat protein); a detectable agent; a pharmaceutical agent; and/or a protein or peptide that can mediate association of the antibody or antibody portion with another molecule (such as a streptavidin core region or a polyhistidine tag). For example, an antibody or antibody portion of the invention can be functionally linked by any suitable method (e.g., chemical coupling, genetic fusion, noncovalent association or otherwise) to one or more other molecular entities. Examples of linkers capable of being used to couple an immunotoxin to an antibody or antibody portion of the invention include, for example, N-succinimidyl 3-(2-pyridyldithio)proprionate (also known as N-succinimidyl 4-(2-pyridyldithio)pentanoate or SPP); 4-succinimidyl-oxycarbonyl-a-(2-pyridyldithio)-toluene (SMPT); N-succinimidyl-3-(2-pyridyldithio)butyrate (SDPB); 2-iminothiolane; S-acetylsuccinic anhydride; disulphide benzyl carbamate; carbonate; hydrazone linkers; N-(α-Maleimidoacetoxy)succinimide ester; N-[4-(p-Azidosalicylamido)butyl]-3'-(2'-pyridyldithio)propionamide (AMAS); N-[β-Maleimidopropyloxy]succinimide ester (BMPS); [N-e-Maleimidocaproyloxy]succinimide ester (EMCS); N-[g-Maleimidobutyryloxy]succinimide ester (GMBS); Succinimidyl-4-[N-Maleimidomethyl]cyclohexane-1-carboxy-[6-amidocaproate] (LC-SMCC); Succinimidyl 6-(3-[2-pyridyldithio]-propionamido)hexanoate (LC-SPDP); m-Maleimidobenzoyl-N-hydroxysuccinimide ester (MBS); N-Succinimidyl[4-iodoacetyl]aminobenzoate (SlAB); Succinimidyl 4-[N-maleimidometbyl]cyclohexane-1-carboxylate (SMCC); N-Succinimidyl 3-[2-pyridyldithio]-propionamido (SPDP); [N-e-Maleimidocaproyloxy]sulfosuccinimide ester (Sulfo-EMCS); N-[g-Maleimidobutyryloxy]sulfosuccinimide ester (Sulfo-GMBS); 4-Sulfosuccinimidyl-6-methyl-a-(2-pyridyldithio)toluamido]hexanoate) (Sulfo-LC-SMPT); Sulfosuccinimidyl 6-(3'-[2-pyridyldithio]-propionamido)hexanoate (Sulfo-LC-SPDP); m-Maleimidobenzoyl-N-hydroxysulfosuccinimide ester (Sulfo-MBS); N-Sulfosuccinimidyl[4-iodoacetyl]aminobenzoate (Sulfo-SIAB); Sulfosuccinimidyl 4-[N-maleirnidomethyl]cyclohexane-1-carboxylate (Sulfo-SMCC); Sulfosuccinimidyl 4-[p-maleimidophenyl]butyrate (Sulfo-SMPB); EGS; DST; DOTA; DTPA; and thiourea linkers.

In connection with immunoconjugates, the provided antibodies or antigen binding fragments thereof of the invention can be modified to act as immunoconjugates utilizing techniques that are well known in the art; see e.g. US 5,194,594. In connection with the preparation of radiolabelled antibodies, such modified antibodies can also be readily prepared utilizing techniques that are well known in the art; see e.g. US 4,681,581, US 4,735,210, US 5,101,827, US 5,102,990, US 5,648,471, and US 5,697,902.

As discussed, the antibody or antigen binding fragment thereof of the invention can be conjugated to a therapeutic agent. Examples of therapeutic agents include, but are not limited to, cytochalasin B, gramicidin D, ethidium bromide, emetine, etoposide, tenoposide, colchicin, dihydroxy anthracin dione, mitoxantrone, 1-dehydrotestosterone, glucocorticoids, procaine, tetracaine, lidocaine, propranolol, antimetabolites (e.g., methotrexate, 6-mercaptopurine, 6-thioguanine, cytarabine, 5-fluorouracil decarbazine), alkylating agents (e.g., mechlorethamine, thioepa chlorambucil, CC-1065(see e.g. US 5,475,092, US 5,585,499, US 5,846,545), melphalan, carmustine (BSNU) and lomustine (CCNU), cyclothosphamide, busulfan, dibromomannitol, streptozotocin, granulysin (GNLY), mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, mitomycin, puromycin anthramycin (AMC)), duocarmycin and analogs or derivatives thereof, and anti-mitotic agents (e.g., vincristine, vinblastine, taxol, auristatins (e.g., auristatin E) and maytansinoids, and analogs or homologs thereof.

The conjugates of the invention can be used for modifying a given biological response. The therapeutic agent is not to be construed as limited to classical chemical therapeutic agents. For example, the therapeutic agent may be a protein or polypeptide possessing a desired biological activity. Such proteins may include, for example, a toxin such as abrin, ricin A, pseudomonas exotoxin, gelonin, diphtheria toxin, or a component thereof (e.g., a component of pseudomonas exotoxin is PE38); a protein such as tumor necrosis factor, interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors. Similarly, the therapeutic agent can be a viral particle, e.g., a recombinant viral particle, that is conjugated (e.g., via a chemical linker) or fused (e.g., via a viral coat protein) to an antibody of the invention.

Active radioisotopes can also be coupled to antibodies or antigen binding fragments thereof of the invention. Radioactive isotopes can be used in diagnostic or therapeutic applications. Radioactive isotopes that can be coupled to the antibodies include, but are not limited to α-, β-, or γ-emitters, or β- and γ-emitters. Such radioactive isotopes include, but are not limited to iodine (¹³¹I or ¹²⁵I), yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), indium (¹¹¹In), technetium (^{99m}Tc), phosphorus (³²P), rhodium (⁸⁸Rh), sulfur (³⁵S), carbon (¹⁴C), tritium (³H), chromium (⁵¹Cr), chlorine (³⁶Cl), cobalt (⁵⁷Co or ⁵⁸Co), iron (⁵⁹Fe), selenium (⁷⁵Se), or gallium (⁶⁷Ga). Radioisotopes useful as therapeutic agents include yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), and rhodium (¹⁸⁸Rh). Radioisotopes useful as labels, e.g., for use in diagnostics, include iodine (¹³¹I or ¹²⁵I), indium (¹¹¹In), technetium (^{99m}Tc), phosphorus (³²P), carbon (¹⁴C), and tritium (³H), or one or more of the therapeutic isotopes listed above.

Useful imaging agents with which an antibody or an antibody portion of the invention may be labelled include fluorescent compounds, various enzymes, prosthetic groups, luminescent materials, bioluminescent materials, fluorescent emitting metal atoms, e.g., europium (Eu), and other Lanthanides, and radioactive materials (described above). Exemplary fluorescent detectable agents include fluorescein, fluorescein isothiocyanate, rhodamine, 5-dimethylamine-1-napthalenesulfonyl chloride, phycoerythrin and the like. An antibody may also be derivatized with detectable enzymes, such as alkaline phosphatase, horseradish peroxidase, b-galactosidase, acetylcholinesterase, glucose oxidase and the like. When an antibody is derivatized with a detectable enzyme, it is detected by adding additional reagents that the enzyme uses to produce a detectable reaction product. For example, when the detectable agent horseradish peroxidase is present, the addition of hydrogen peroxide and diaminobenzidine leads to a coloured reaction product, which is detectable. An antibody may also be derivatized with a prosthetic group (e.g., streptavidin/biotin and avidin/biotin). For example, an antibody may be derivatized with biotin, and detected through indirect measurement of avidin or streptavidin binding. Examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; and examples of bioluminescent materials include luciferase, luciferin, and aequorin.

### Pharmaceutical Compositions:

In another aspect, the present invention provides compositions, e.g., pharmaceutically acceptable compositions, which include an antibody or an antigen binding fragment thereof of the invention formulated together with a pharmaceutically acceptable carrier.

As used herein, "pharmaceutically acceptable carrier" includes any and all solvents, dispersion media, isotonic and absorption delaying agents, and the like that are physiologically compatible. The carrier can be suitable for intravenous, intramuscular, subcutaneous, parenteral, rectal, spinal or epidermal administration (e.g., by injection or infusion).

The compositions of this invention may be in a variety of forms. These include, for example, liquid, semi-solid and solid dosage forms, such as liquid solutions (e.g., injectable and infusible solutions), dispersions or suspensions, liposomes and suppositories. The preferred form depends on the intended mode of administration and therapeutic application. Typical compositions are in the form of injectable or infusible solutions. The mode of administration is parenteral (e.g., intravenous, subcutaneous, intraperitoneal, intramuscular). In some embodiments, the antibody is administered by intravenous infusion or injection. In other embodiments, the antibody is administered by intramuscular or subcutaneous injection.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Therapeutic compositions typically should be sterile and stable under the conditions of manufacture and storage. The composition can be formulated as a solution, microemulsion, dispersion, liposome, or other ordered structure suitable to high antibody concentration. Sterile injectable solutions can be prepared by incorporating the active compound (i.e., antibody or antibody portion) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the provided methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatine.

The antibodies and antigen binding fragments of the present invention can be administered by a variety of methods known in the art, although for many therapeutic applications, the route/mode of administration is intravenous injection or infusion. As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are generally known to those skilled in the art.

In certain embodiments, an antibody or an antibody portion of the invention may be orally administered, for example, with an inert diluent or an assimilable edible carrier. The compound (and other ingredients if desired) may also be enclosed in a hard- or soft-shell gelatine capsule, compressed into tablets, buccal tablets, troches, capsules, elixirs, suspensions, syrups, wafers, and the like. To administer an antibody or an antibody fragment of the invention by other than parenteral administration, it may be necessary to coat the compound with, or co-administer the compound with, a material to prevent its inactivation.

The pharmaceutical compositions of the invention can be administered with medical devices known in the art.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

An exemplary, non-limiting range for a therapeutically or prophylactically effective amount of an antibody or an antigen binding fragment of the invention is 0.1-20 mg/kg, or 1-10 mg/kg. It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

The pharmaceutical compositions of the invention may include a "therapeutically effective" amount of an antibody or an antigen binding fragment of the invention. A "therapeutically effective" amount refers to an amount effective, at dosages and for periods of time necessary, to achieve the desired therapeutic result. A therapeutically effective amount of the modified antibody or antibody fragment may vary according to factors such as the disease state, age, sex, and weight of the individual, and the ability of the antibody or antibody portion to elicit a desired response in the individual. A therapeutically effective amount is also one in which any toxic or detrimental effects of the modified antibody or antibody fragment is outweighed by the therapeutically beneficial effects. A "therapeutically effective dosage" preferably inhibits a measurable parameter, e.g., tumor growth rate by at least about 20%, at least about 40%, at least about 60%, and in some aspects preferably at least about 80% relative to untreated subjects. The ability of a compound to inhibit a measurable parameter, e.g., cancer, can be evaluated in an animal model system predictive of efficacy in human tumors. Alternatively, this property of a composition can be evaluated by examining the ability of the compound to inhibit, such inhibition *in vitro* by assays known to the skilled practitioner.

Also within the scope of the invention are kits comprising an antibody or antigen-binding fragment thereof of the invention. Also included in the invention are kits comprising immunoconjugates comprising an antibody or antigen binding fragment conjugated to an active agent. Further included are kits comprising liposome compositions comprising antibodies or antigen binding fragments thereof of the invention. The kit can include one or more other elements including: instructions for use; other reagents, e.g., a label, a therapeutic agent, or an agent useful for chelating, or otherwise coupling, an antibody to a label or therapeutic agent, or a radioprotective composition; devices or other materials for preparing the antibody for administration; pharmaceutically acceptable carriers; and devices or other materials for administration to a subject. Instructions for use can include instructions for diagnostic applications of the antibodies or antigen-binding fragment thereof to detect human NPTXR, *in vitro,* e.g., in a sample, e.g., a biopsy or cells from a subject, or *in vivo.* The instructions can include instructions for therapeutic application including suggested dosages and/or modes of administration in a subject. Other instructions can include instructions on coupling of the antibody to a chelator, a label or a therapeutic agent, or for purification of a conjugated antibody, e.g., from unreacted conjugation components. As discussed above, the kit can include a label or imaging agent, e.g., any of the labels described herein. As discussed above, the kit can include a therapeutic agent, e.g., a therapeutic agent described herein. In some applications the antibody will be reacted with other components, e.g., a chelator or a label or therapeutic agent, e.g., a radioisotope, e.g., yttrium or lutetium. In such cases the kit can include one or more of a reaction vessel to carry out the reaction or a separation device, e.g., a chromatographic column, for use in separating the finished product from starting materials or reaction intermediates.

The kit can further contain at least one additional reagent, such as a diagnostic or therapeutic agent, e.g., a diagnostic or therapeutic agent as described herein, and/or one or more additional antibodies (or fragments thereof), formulated as appropriate, in one or more separate pharmaceutical preparations.

### Uses of the Invention:

The antibodies have *in vitro* and *in vivo* diagnostic, therapeutic and prophylactic utilities. For example, these antibodies can be administered to cells in culture, e.g. *in vitro* or ex vivo, or in a subject, e.g., *in vivo,* to treat, prevent, and/or diagnose a variety of disorders, disease or medical condition. More specifically, antibodies or antigen binding fragments thereof of the invention and compositions comprising the provided antibodies or antigen binding fragments thereof (e.g., immunoconjugates), can be used for diagnosis or treatment of stomach cancer, colorectal cancer, liver cancer, bladder cancer, breast cancer, esophageal cancer, glioblastoma, glioma, lung cancer, malignant melanoma, ovarian cancer, cervical cancer, malignant pheochromocytoma, prostatic cancer, testicular germ cell tumor, thyroid cancer, pancreatic cancer, uterine cancer, and renal cell cancer, more preferably liver cancer, bladder cancer, thyroid cancer, preferably of liver cancer, bladder cancer, thyroid cancer, stomach cancer, colorectal cancer, breast cancer, lung cancer, pancreatic cancer, and/or oesophageal cancer, more preferably of stomach cancer, colorectal cancer, breast cancer, lung cancer, pancreatic cancer, and/or oesophageal cancer.

As used herein, the term "subject" is intended to include human and non-human animals. For example, a subject includes a patient (e.g., a human patient, a veterinary patient), having a disorder to be diagnosed, prevented or treated using the antibody or antigen binding fragment thereof of the invention. In one embodiment, the subject is a human subject.

The antibodies or antigen-binding fragments thereof of the invention may be used in combination with other therapies. For example, the combination therapy can include a composition of the present invention co-formulated with, and/or co-administered with, one or more additional therapeutic agents. In other embodiments, the antibodies are administered in combination with other therapeutic treatment modalities, including surgery, radiation, cryosurgery, and/or thermotherapy. Such combination therapies may advantageously utilize lower dosages of the administered therapeutic agents, thus avoiding possible toxicities or complications associated with the various monotherapies.

Labelled antibodies can be used, for example, diagnostically and/or experimentally in a number of contexts, including (i) to isolate a predetermined antigen by standard techniques, such as affinity chromatography or immunoprecipitation; (ii) to detect a predetermined antigen (e.g., in a cellular lysate or cell supernatant) in order to evaluate the abundance and pattern of expression of the protein; (iii) to monitor protein levels in tissue as part of a clinical testing procedure, e.g., to determine the efficacy of a given treatment regimen.

Thus, in another aspect, the present invention provides a diagnostic method for detecting the presence of human NPTXR *in vitro* (e.g., in a biological sample, such as a tissue biopsy). The method includes: (i) contacting the sample with an antibody or antigen binding fragment thereof of the invention, or administering to the subject, the antibody or antigen binding fragment thereof of the invention; (optionally (ii) contacting a reference sample, e.g., a control sample (e.g., a control biological sample, such as plasma, tissue, biopsy, or a control subject)); and (iii) detecting formation of a complex between the antibody or antigen binding fragment thereof of the invention and the sample or subject, or the control sample or subject, wherein a change, e.g., a statistically significant change, in the formation of the complex in the sample or subject relative to the control sample or subject is indicative of the presence of human NPTXR in the sample.

Preferably, the antibody or antigen binding fragment thereof of the invention is directly or indirectly labelled with a detectable substance to facilitate detection of the bound or unbound antibody. Suitable detectable substances include various enzymes, prosthetic groups, fluorescent materials, luminescent materials and radioactive materials, as described above and described in more detail below.

Complex formation between the antibody and the antigen can be detected by measuring or visualizing either the antibody (or antibody fragment) bound to the antigen or unbound antibody (or antibody fragment). Conventional detection assays can be used, e.g., an enzyme-linked immunosorbent assays (ELISA), a radioimmunoassay (RIA) or tissue immunohistochemistry. Alternative to labelling the antibody, the presence of the antigen can be assayed in a sample by a competition immunoassay utilizing standards labelled with a detectable substance and an unlabelled antibody. In this assay, the biological sample, the labelled standards and the antigen binding agent are combined and the amount of labelled standard bound to the unlabelled antibody is determined. The amount of antigen in the sample is inversely proportional to the amount of labelled standard bound to the antigen binding agent. Furthermore, the antibody may be used for diagnosis e.g. via fluorescence guided biopsies and as supportive tool in fluorescence guided surgeries.

### FIGURES:

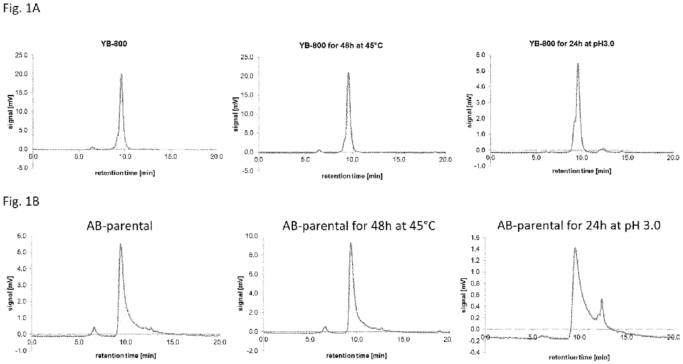

- Fig. 1:: shows the result of an analytical HPLC-SEC with and without stress induction for YB-800 (Fig. 1A) and AB-parental (Fig. 1B); wherein YB-800 shows a well-established monomeric peak not only under temperature stress but also under low pH stress whereas AB-parental shows significant signs of degradation under low pH stress.

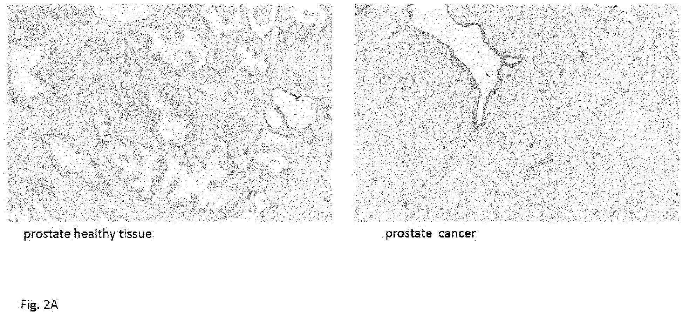

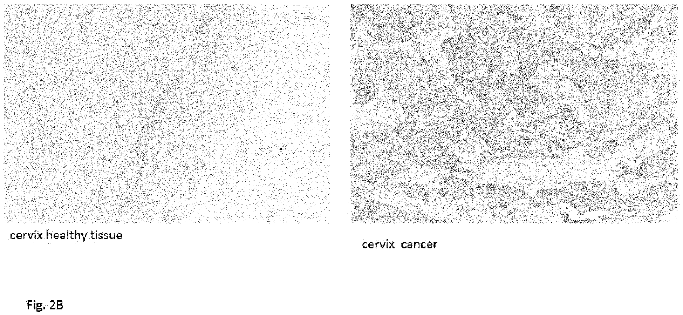

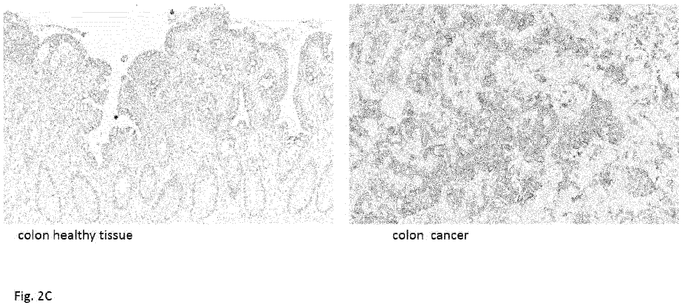

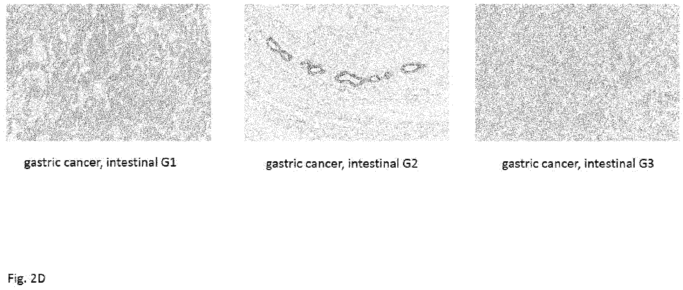

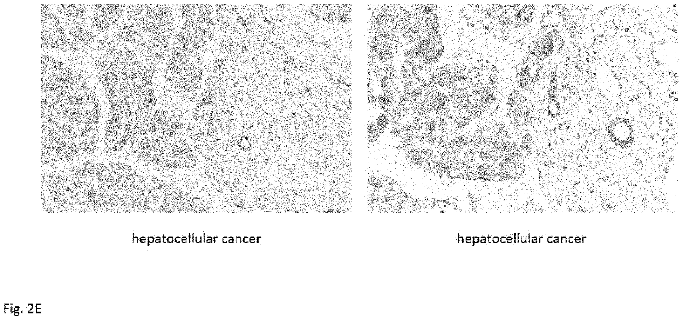

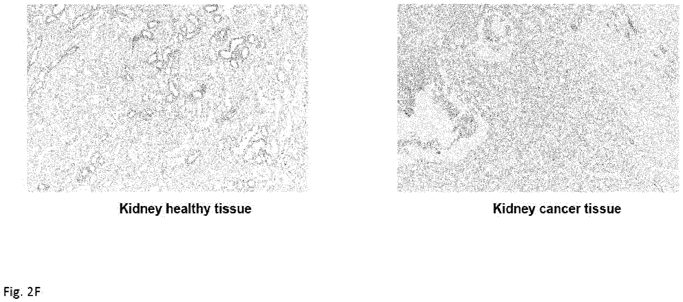

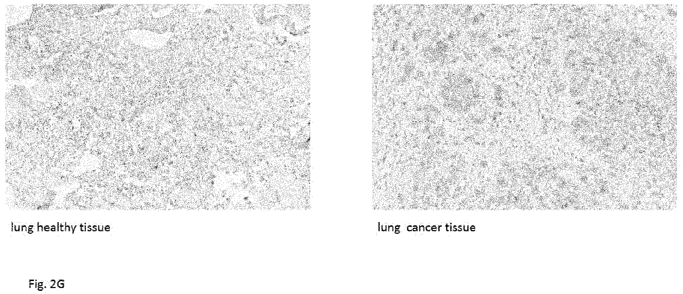

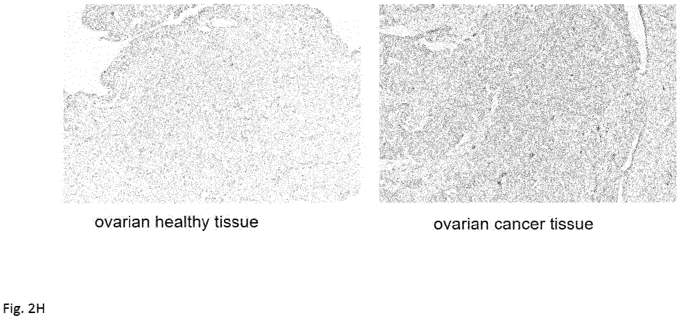

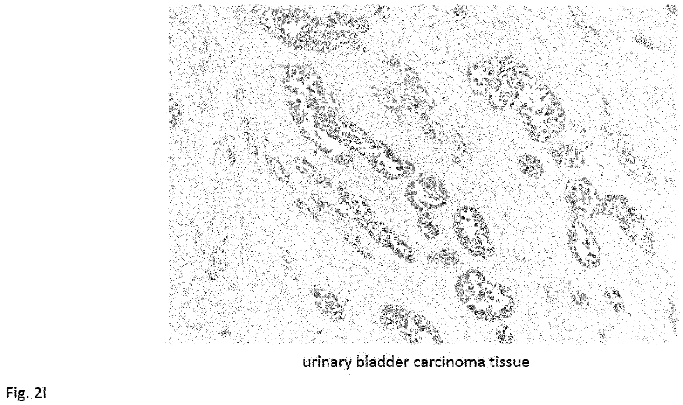

- Fig. 2:: shows the results of DAB Immunohistochemistry YB-800 (Anti-NPTxR_VH1_VK1) on human tumor and normal tissue; Fig. 2A shows positive YB-800 staining in prostate cancer tissue (right panel) compared to prostate healthy tissue (left panel); Fig. 2B shows positive YB-800 staining in cervix cancer tissue (right panel) compared to cervix healthy tissue (left panel); Fig. 2C shows positive YB-800 staining in colon cancer tissue (right panel) compared to colon healthy tissue (left panel); Fig. 2D shows positive YB-800 staining in gastric cancer tissue at various stages with G1 (left panel), G2 (middle panel) and G3 (right panel); Fig. 2E shows positive YB-800 staining in hepatocellular cancer tissue at a first magnification (left panel) and a second magnification (left panel); Fig. 2F shows positive YB-800 staining in kidney cancer tissue (right panel) compared to kidney healthy tissue (left panel); Fig. 2G shows positive YB-800 staining in lung cancer tissue (right panel) compared to lung healthy tissue (left panel); Fig. 2H shows positive YB-800 staining in ovarian cancer tissue (right panel) compared to ovarian healthy tissue (left panel); Fig. 2I shows positive YB-800 staining in urinary bladder carcinoma tissue.

In the following the invention is explained in more detail by way of examples.

### EXAMPLES:

### Example 1: Antibody production

For the purpose of the present invention, an antibody according to the invention is compared to the prior art antibody specific for NPTXR as disclosed in EP 3 744 346 A1.

The antibody of the invention, denoted herein YB-800, differs from the so-called parental antibody (AB-parent), which comprises the CDR sequences of the prior art antibody binding specifically to NPTXR as disclosed in EP 3 744 346 A1, by amino acid changes in the CDRs HCDR2 and HCDR3 as set forth below.

YB-800 comprises three heavy chain complementarity determining regions (HCDR1, HCDR2, and HCDR3), and three light chain complementarity determining regions (LCDR1, LCDR2, and LCDR3), wherein HCDR1 comprises or consists of the amino acid sequence of NYGMN (SEQ ID NO: 4); HCDR2 comprises or consists of the amino acid sequence of WINTDIGEPTYSQKFQG (SEQ ID NO: 5); and HCDR3 comprises or consists of the amino acid sequence of EMDV (SEQ ID NO: 6); and wherein LCDR1 comprises or consists of the amino acid sequence of RASQSISDYLH (SEQ ID NO: 7); LCDR2 comprises or consists of the amino acid sequence of YVSHSIS (SEQ ID NO: 8); and LCDR3 comprises or consists of the amino acid sequence of LNGHSFPFT (SEQ ID NO: 9).

AB-parental comprises three heavy chain complementarity determining regions (HCDR1, HCDR2, and HCDR3), and three light chain complementarity determining regions (LCDR1, LCDR2, and LCDR3), wherein HCDR1 comprises or consists of the amino acid sequence of NYGMN (SEQ ID NO: 4); HCDR2 comprises or consists of the amino acid sequence of WINTDIGEPTY**AEE**F**K**G (SEQ ID NO: 15); and HCDR3 comprises or consists of the amino acid sequence of EMD**S** (SEQ ID NO: 16); and wherein LCDR1 comprises or consists of the amino acid sequence of RASQSISDYLH (SEQ ID NO: 7); LCDR2 comprises or consists of the amino acid sequence of YVSHSIS (SEQ ID NO: 8); and LCDR3 comprises or consists of the amino acid sequence of LNGHSFPFT (SEQ ID NO: 9). Differences in amino acid sequence to CDRs of YB-800 are highlighted in bold and underlined.

Antibody variants were cloned into mammalian expression vectors, containing either the constant domains of the human IgG1 heavy chain or light chain respectively. Transfection grade plasmid DNA was generated and verified by DNA sequence analysis. The plasmid DNA was used for transient transfection of mammalian cells. After one week of antibody production in mammalian cell culture, the antibodies were purified. After clearance of the culture supernatant from cells by centrifugation, the IgG antibodies were purified by protein A affinity chromatography. The final buffer of the purified antibodies is phosphate buffered saline (no additive included). The protein concentration of the antibodies was determined by UV/VIS spectrometry at 280 nM. Integrity and purity of the antibodies was assessed by SDS-PAGE under reducing conditions and analytical HPLC-SEC analysis. The functional binding activity of the antibodies to the target antigen was measured by ELISA.

### Example 2: Antibody binding

The antibodies, YB-800 and AB-parent, were tested by ELISA for antibody binding to the positive antigen, recombinant human NPTXR (rhNPTXR; Ala24 to Ala500 of Acc no. #O95502) immobilized to an ELISA plate. Additionally, a peptide (mNPTXR) representing the mouse NPTXR epitope space, said peptide consisting of an amino acid sequence of CESGLPRGLQDAGPRRDT (SEQ ID NO: 14), was immobilized via streptavidin capturing to the ELISA plate. For control of unspecific binding streptavidin was used. A titration series (1/square(10)) of each antibody starting at 10 µg/ml was performed and incubated on the coated and blocked ELISA plates. For detection, a HRP coupled secondary antibody was used (Anti-Human IgG (Fc specific) - Peroxidase antibody; Sigma Aldrich #A0170). Bound antibodies were detected using TMB reaction, followed by absorbance reading at 450 nm. Using the ELISA absorbance an EC50 value was calculated.

The results are summarized in Table 1:

**Table 1:**

| antibody | antigen | EC50 [ng/ml] | 95% CI EC50 [ng/ml] | R squared |
|---|---|---|---|---|
| YB-800 | rhNPTXR | 27.13 | 23.54 to 31.28 | 0.9971 |
| YB-800 | Strep + Bio-mNPTXR | 30.86 | 23.31 to 41.08 | 0.9898 |
| AB-parent | rhNPTXR | 42.17 | 38.62 to 46.04 | 0.9988 |
| AB-parent | Strep + Bio-mNPTXR | 93.39 | 76.30 to 114.6 | 0.9943 |

YB-800 exhibits a lower EC50 for both, human and mouse NPTXR compared to the parental antibody AB-parental. Further, the EC50 of YB-800 is comparable for both human (27.13) and mouse (30.86) NPTXR, while the EC50 for AB-parental differs significantly between human NPTXR (42.17) and mouse NPTXR (93.39). Thus, results which have been generated with YB-800 on mouse specimens will translate better to the corresponding human situation than results which have been generated using AB-parental. Therefore, YB-800 has improved suitability as research tool as well as medical tool compared to AB-parental.

### Example 3: Analytical HPLC-SEC with and without stress induction

Antibodies YB-800 and AB-parental were characterized by analytical HPLC-SEC analysis to check antibody aggregation and degradation before and after stress induction.

Three samples of each antibody were prepared with a concentration of 0.2 mg/ml. The control sample was incubated 48 hours at 4°C whereas the temperature stress sample was incubated 48 hours at 48°C. The third sample was incubated in a pH 3.0 buffer for 24 hours. After 24 hours at pH 3.0 the buffer was exchanged to PBS at pH 7.4 before performing analytical HPLC-SEC. All three samples were used for direct comparison in analytical HPLC-SEC.

The results are summarized in Figs 1A and 1B as well as in Table 2 below.

As shown in Fig. 1A, YB-800 showed nice monomeric peak in the control sample and no significant degradation or aggregation. Even at elevated temperature (45°C) or pH-stress (low pH 3) YB-800 remained stable and showed no degradation or aggregation.

As shown in Fig. 1B, the control sample of the parental antibody AB-parental showed already a measurably broader peak, which did not substantially change at temperature stress, but after pH 3.0 for 24 hours the AB-parental antibody showed significant degradation indicating poor stability.

**Table 2:**

| **Sample** | **Retention time [min]** | **Area [%]** |
|---|---|---|
| YB-800 | 9.609 | 96.655 |
| YB-800 48h at 45°C | 9.600 | 96.310 |
| YB-800 24h at pH 3 | 9.604 | 100.00 |
| AB-parental | 9.468 | 95.559 |
| AB-parental 48h at 45°C | 9.371 | 95.483 |
| AB-parental 24h at pH 3 | 9.569 | 78.824 |

### Example 4: FACS analysis of human cancer cell lines by using YB-800

### Disposables and instruments:

FACS machine (Beckman CytoFlex S, Equip ID: ZAFCM0020); Centrifuge (Eppendorf 5910R, Equip ID: ZACEN0110); and Cell counter (Nexcelom Bioscience Cellometer^{®} Auto T4, Equip ID: ZAPUS0030).

### Reagents:

FBS (absin, CAT# abs49001013b); EDTA (Invitrogen^{™}, CAT#15575020); Human BD Fc Block^{™} (BD Biosciences, cat#564220); PBS sterile (Cellmax, CAT# CBS101.05); UltraComp eBeads (eBioscience CAT#01-2222-42); FACS Buffer ∅ 1 × PBS, 0.1% BSA, 2mM EDTA.

### FACS antibody information:

**Table 3. FACS Antibodies information**

| **Markers** | **Fluorochrome** | **Isotype** | **Cat.** | **Conc. of Ab** | **Abl reaction** | **Vender** |
|---|---|---|---|---|---|---|
| Target antibody-YB-800 (1st antibody) | - | Human IgG | YB-800 (anti-NPTxR_VH1 _VK1) | 5 mg/ml | 10 µg/ml | Client |
| Goat Anti-human IgG Fc (2nd antibody) | PE | polyclonal | 12-4998-82 | 0.5 mg/ml | 10 µg/ml | Invitrogen |
| Live and Dead | APC-eFluor780 | - | 65-0865-14 | - | 1 | eBioscience |

**Table 4. Tube Information for Each Sample**

| **Tube name** | **primary antibody** | | **secondary antibody** | **L/D** |
|---|---|---|---|---|
| | **Target antibody** | **(Isotype)** | | |
| Sample | + YB-800 (working conc.c 10 ug/mL) | - | + Goat Anti-human IgG Fc (working con.c 10 µg/ml) | + |
| Negtive control-blank | - | - | - | + |
| Negtive control-2^{nd} only | - | - | + Goat Anti-human IgG Fc (working con.c 10 µg/ml) | + |

### Procedure:

### Test Article Processing:

1 vial of test articles was dissolved and aliquoted to 100 µL/tube after they arrived; to make sure the antibody was thawed only once per application at CrownBio.

FACS staining for cell lines:
1. 1 × 106 cells were transferred to labelled FACS tube.
2. YB-800 and UD were added to each sample (final staining volume of 100 µL to minimize any small pipetting) with PBS containing 2% FBS.
3. The cells were vortexed gently to mix and stained for 30 min at 4°C in the dark.
4. 2 mL FACS Buffer was added to each tube, and the cells were re-suspended gently. The tubes were centrifuged at 300×g for 5 min, and the supernatant was discarded.
5. Step 4 was repeated one more time.
6. The secondary antibody was diluted in FACS Staining Buffer and 100 µL diluted secondary antibody were added to the cells. Each tube was stained at 4°C for 30 min in the dark.
7. 2 mL FACS Buffer was added to each tube and the cells were re-suspended gently. The tubes were centrifuged at 300×g for 5 min, and the supernatant was discarded.
8. Step 7 was repeated one more time.
9. The samples were analyzed on cytometer.

### Results:

FACS analyses on various tumor cell lines were conducted, which turned out all positive:

| **Cell Line** | **Organ** |
|---|---|
| huH-7 | Liver |
| JHH-4 | Liver |
| SNU-475 | Liver |
| TT | Thyroid Gland |
| Hs578T | Female Breast |
| T47D | Female Breast |
| MCF10A | Female Breast |
| OE19 | Stomach |
| KYSE-150 | Stomach |
| MIA PaCa-2 | Pancreas |
| BxPC-3 | Pancreas |
| CaPan-1 | Pancreas |
| DLD-1 | Colon |
| HCT 116 | Colon |
| HT-29 | Colon |
| LoVo | Colon |
| SW-480 | Colon |
| SW-620 | Colon |

### Example 5: DAB Immunohistochemistry YB-800 (Anti-NPTxR_VH1_VK1) on human tumor and normal tissue

### Method and Materials:

Immunohistochemistry analysis of different human tumor and normal tissues using YB-800 as primary antibody was carried out using the following methodology on Paraffin embedded human tissue. The tissue was sliced using a Manual Rotary Microtome (Leica RM2235) and moved onto Microscope slides, SuperFrost^{®} plus for further processing:

### Day 1

| | | |
|---|---|---|
| Paraffine removal: | Xylol | 3 × 10min |
| | 100%, 96%, 70% Ethyl Alcohol | each for 5 min |
| | Aqua dest | 5 min |
| Demasking: | Citrate Buffer pH 6,0 (1,05g Citric Acid/500ml Aqua dest) Heat to 95°C | 30 min |
| | Cool down in Citrate Buffer | 15 min |
| | Rinse in PBS | rinse |
| Permeabilization: | 0,1% Triton in PBS | 10 min |
| | Rinse in PBS | rinse |
| Blocking: | 1% BSA in PBS + 5% IgG Host of second AB (Goat) | 60 min at room temp |
| YB-800: | Antibody 1:1000 in 1% BSA in PBS | overnight at 4°C |

### Day 2

| | | |
|---|---|---|
| | PBS | rinse + 2 × 10 min |
| Second Antibody: | Goat-anti-Human 1:500 in 1% BSA in PBS | 60 min at RT |
| | ABC-Complex: prepare 30 min before usage | |
| | PBS | 2 × 10 min |
| ABC-Complex: | 1µl A + 1µl B + 250µl 1% BSA in PBS | 30 min RT |
| | Rinse in PBS | rinse |
| | TBS | 10 min |
| DAB: control | prepare according to protocol and apply drop-wise | 0.5-1 min visual |
| | Rinse in PBS | rinse |
| Counter Staining: | Rinse in Aqua dest | 5 min |
| | Hematoxylin | 3 min |
| | Rinse in Aqua. dest | 5 min |
| | 0,5% HCL in Ethyl Alcohol | rinse quickly (3 sec) |
| | Tap Water - develop blue dye | 10 min |
| | Aqua dest | rinse |
| Dehydration: | 70%, 96%, 100% Ethyl Alcohol | 5 min each |
| Xylol | | 2x 10 min |
| Cover under Xylol | | |

### Results:

A positive reaction on tumor tissue could be detected in the following organs:
- Lung Cancer
- Colorectal Cancer
- Cervix Cancer
- Ovarian Cancer
- Gastric Cancer
- Bladder Cancer
- Hepatocellular Cancer
- Kidney Cancer
- Prostate Cancer

## Claims

1. An isolated antibody or antigen binding fragment thereof binding specifically to human NPTXR with the amino acid sequence of SQ ID NO: 1, wherein the isolated antibody or antigen binding fragment comprises three heavy chain complementarity determining regions (HCDR1, HCDR2, and HCDR3), and three light chain complementarity determining regions (LCDR1, LCDR2, and LCDR3), wherein
HCDR1 comprises the amino acid sequence of SEQ ID NO: 4;
HCDR2 comprises the amino acid sequence of SEQ ID NO: 5; and
HCDR3 comprises the amino acid sequence of SEQ ID NO: 6; and wherein
LCDR1 comprises the amino acid sequence of SEQ ID NO: 7;
LCDR2 comprises the amino acid sequence of SEQ ID NO: 8; and
LCDR3 comprises the amino acid sequence of SEQ ID NO: 9.

2. The isolated antibody or antigen binding fragment thereof of any one of the preceding claims, wherein the antibody or antigen binding fragment specifically binds to a peptide consisting of an amino acid sequence of CESGLPRGLQDAGPRRDT (SEQ ID NO: 14), or of GLPRGLQGAGPRRDT (SEQ ID NO: 2), or of CESGLPRGLQGAGPRRDT (SEQ ID NO: 3).

3. The isolated antibody or antigen binding fragment thereof of any one of the preceding claims, wherein the antibody or antigen binding fragment comprises a heavy chain V_{H} comprising the amino acid sequence of SEQ ID NO: 10, and a light chain V_{L} comprising the amino acid sequence of SEQ ID NO: 11.

4. The isolated antibody or antigen binding fragment thereof of any one of the preceding claims, wherein the antibody is monoclonal.

5. The isolated antibody or antigen binding fragment thereof of any one of the preceding claims,
wherein the antibody is recombinant; or
wherein the antibody is an IgG, IgM, IgA or an antigen binding fragment thereof; or
wherein the antibody is a Fab fragment, a Fab' fragment, a F(ab')2 fragment, a F(ab')3 fragment, a Fd fragment, a Fd' fragment, a Fv fragment, a scFv, a bivalent scFv, a diabody, a linear antibody, or a monovalent.

6. The isolated antibody or antigen binding fragment thereof of any one of the preceding claims, wherein the antibody is a humanized antibody or de-immunized antibody.

7. The isolated antibody or antigen binding fragment thereof of any one of the preceding claims, wherein the antibody is conjugated to an active agent, preferably said active agent comprises an imaging agent, a therapeutic agent, a toxin and/or a radionuclide.

8. A pharmaceutical composition comprising the isolated antibody or antigen binding fragment thereof of any one of claims 1 to 7 and a pharmaceutically or physiologically acceptable carrier or excipient.

9. A polynucleotide molecule comprising a nucleic acid sequence encoding an isolated antibody or antigen binding fragment thereof of any one of claims 1 to 7.

10. The polynucleotide molecule of claim 9, wherein the polynucleotide molecule comprises a nucleic acid sequence with SEQ ID NO: 12 and/or a nucleic acid sequence with SEQ ID NO: 13.

11. A host cell comprising one or more polynucleotide molecule(s) of any one of claims 9 or 10, optionally wherein the host cell is a mammalian cell, a yeast cell, a bacterial cell, a ciliate cell or an insect cell.

12. A method of manufacturing an antibody comprising:
(a) expressing one or more polynucleotide molecule(s) encoding an isolated antibody or antigen binding fragment thereof of any one of claims 1 to 7 in a cell; and
(b) purifying the antibody from the cell.

13. A kit comprising an isolated antibody or antigen binding fragment of any of claims 1 to 7 and/or a polynucleotide molecule of any one of claims 9 or 10, and instructions for use of the antibody or polynucleotide molecule, optionally further wherein the antibody or polynucleotide molecule is lyophilized.

14. An isolated antibody or antigen binding fragment of any of claims 1 to 7, or a pharmaceutical composition of claim 8, for use in a diagnostic method of a disease or medical condition, preferably the disease or medical condition is stomach cancer, colorectal cancer, liver cancer, bladder cancer, breast cancer, esophageal cancer, glioblastoma, glioma, lung cancer, malignant melanoma, ovarian cancer, cervical cancer, malignant pheochromocytoma, prostatic cancer, testicular germ cell tumor, thyroid cancer, pancreatic cancer, uterine cancer, and renal cell cancer, more preferably liver cancer, bladder cancer, thyroid cancer, preferably of liver cancer, bladder cancer, thyroid cancer, stomach cancer, colorectal cancer, breast cancer, lung cancer, pancreatic cancer, and/or oesophageal cancer, more preferably of stomach cancer, colorectal cancer, breast cancer, lung cancer, pancreatic cancer, or oesophageal cancer.

15. An isolated antibody or antigen binding fragment of any of claims 1 to 7, or a pharmaceutical composition of claim 8, for use in treating or preventing a disease or medical condition, preferably the disease or medical condition is stomach cancer, colorectal cancer, liver cancer, bladder cancer, breast cancer, esophageal cancer, glioblastoma, glioma, lung cancer, malignant melanoma, ovarian cancer, cervical cancer, malignant pheochromocytoma, prostatic cancer, testicular germ cell tumor, thyroid cancer, pancreatic cancer, uterine cancer, and renal cell cancer, more preferably liver cancer, bladder cancer, thyroid cancer, preferably of liver cancer, bladder cancer, thyroid cancer, stomach cancer, colorectal cancer, breast cancer, lung cancer, pancreatic cancer, and/or oesophageal cancer, more preferably of stomach cancer, colorectal cancer, breast cancer, lung cancer, pancreatic cancer, or oesophageal cancer.
